# EUROPEAN PATENT APPLICATION

(11) **EP 4 467 113 A1**
(43) Date of publication of application: **27.11.2024**
(21) Application number: 23175527.3
(22) Date of filing: 25.05.2023
(51) Int. Cl.: A61F 5/41

(54) **PENIL EPIDERMAL PATCH/RING ERECTION SYSTEM**

(71) Applicant: Gedik, Hikmet Selcuk Megar Lab Ve Test A.S., 06700 Ankara (TR)
(72) Inventor: GEDIK, HIKMET SELÇUK, 06700 Ankara (TR)

(57) **Abstract**

Cross-Section of Adhesive Controlled (TDDS 3 Matrix Diffusion-Controlled Systems) In this approach, the Papaverine reservoir is prepared by homogenously dispersing Papaverine particles in a hydrophilic or lipophilic polymer matrix. The resultant medicated polymer is then moulded into a medicated disc with a defined surface area and controlled thickness. The Papaverine reservoir can be formed by dissolving Papaverine and polymer in a common solvent followed by solvent evaporation in a mould at an elevated temperature and/or vacuum. The Papaverine reservoir containing polymer disc is then pasted onto an occlusive base plate in a compartment fabricated from a Papaverine impermeable plastic backing. The adhesive polymer is then spread along the circumference to from a strip of adhesive rim around the medicated disc.

## Description

The main functions of the penis include reproduction and urination. Penises vary in length, girth, and appearance but mostly all have the same anatomy, which allows them to perform their functions.

The parts of the penis are the base, shaft, glans, and foreskin. The tissues that make up the penis include the dorsal nerve, blood vessels, connective tissue, and erectile tissue (corpus cavernosum and corpus spongiosum).

Papaverine is a substance that expands smooth muscles. In medicine, it is used in cases where the blood vessels in the brain need to be expanded and in men who have erectile dysfunction. It is obtained from the plant Papaver somniferum. It shows its effect by suppressing the phosphodiesterase enzyme at the cellular level. When this enzyme is suppressed, there is an increase in cAMP and cGMP levels in the cell. This results in relaxation of the smooth muscles in the veins or in the penis. Due to this relaxation, hardening (erection) occurs in the penis.

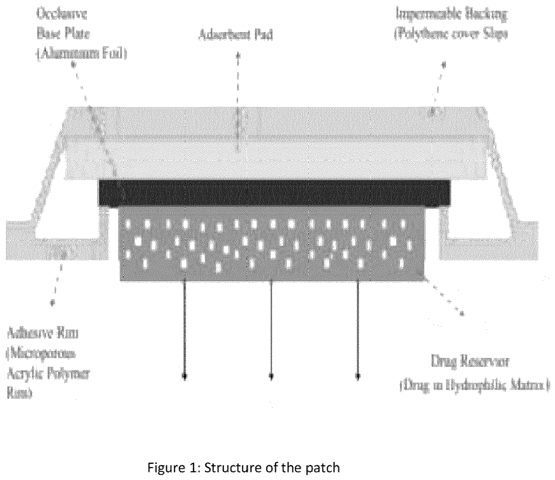

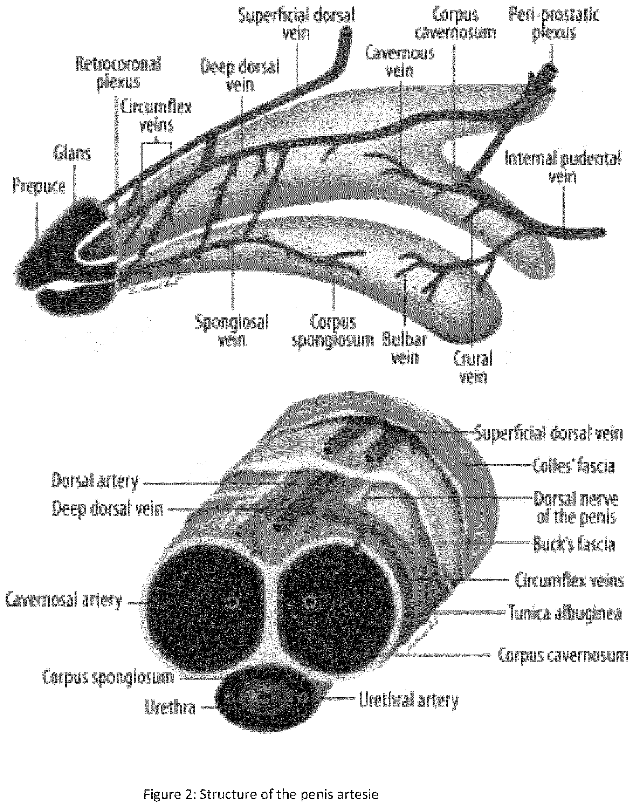

## Claims

1. Our claim is, design of patch/ring for application to use Papaverine to penis more effective method , when the anatomy of the penis is examined, there are arterial structures in the deep and veins in the more superficial part. Our features are that the patch, which has a circular and elastic structure, blocks the venues return when placed on the root of the penis on the one hand. By narrowing that section a little, the venues blocks venous return and causes blood to be trapped in the penis. This is the fundamental mechanism of erection. In other words, on the one hand, when that circular Papaverine reaches the alter structures in the skin, it makes arterial dilation, that is, widens, allowing the artery to carry more blood to the penis, on the other hand, its circular elastic structure shrinks the veins a little, delaying the return of the pumped blood from the penis, and by trapping the blood there, it causes the erection. More and makes the hardening perfect. This is another mechanism of action, and we ensure the maximum effect of the product developed with both a drug pharmacological mechanism and an anatomical physiological mechanism.
Our product advantage is as follows;
Patch/ring benefits;
➢ Slow release compared to injection contributes to control hardening.
➢ The risk of priyasmispus formation is much lower than injection.
➢ There is no risk of injection-related pain and fibrosis development.
On the other hand disadvantages of injection as follows;
➢ Hematomas, systemic side effects,
➢ Penile calcification
➢ This system can be used easily since injections cannot be made in patients with bleeding disorders.
